# EUROPÄISCHE PATENTANMELDUNG

(11) **EP 0 850 645 A2**
(43) Veröffentlichungstag der Anmeldung: **01.07.1998**
(21) Anmeldenummer: 97111291.7
(22) Anmeldetag: 04.07.1997
(51) Int. Cl.: A61K 9/00, A61L 9/03

(54) **Verfahren und Vorrichtung zur Freisetzung von Wirkstoffen aus Heilpräparaten zur Langzeitbehandlung von Erkrankungen der Atemwege**

(30) Priorität: 28.12.1996 DE 19654631
(71) Anmelder: Badewien, Reinhard, 26802 Moormerland (DE)
(72) Erfinder: Badewien, Reinhard, 26802 Moormerland (DE)

(57) **Zusammenfassung**

Zur Freisetzung von Wirkstoffen aus Heilpräparaten zwecks Langzeitbehandlung von Erkrankungen der Atemwege werden die Heilpräparate in ein Reservoir eingegeben und mittels einer thermostatgeregelten Heizeinrichtung auf eine vorbestimmte Temperatur schonend erwärmt und auf der vorbestimmten Temperatur gehalten. Die an die Luft in einem Raum in sehr verträglicher Konzentration aufgrund der Erwärmung freigesetzten Wirkstoffe können ständig von einem im Raum befindlichen Patienten eingeatmet werden. Die thermostatgeregelte Heizeinrichtung kann mit einem Wasserbad arbeiten, in das ein als Kräuterreservoir ausgebildeter Schwimmnapf setzbar ist. Das Kräuterreservoir kann jedoch in einem vorbestimmten Abstand, z.B. mittels eines Ständerteils, über einer Strahlungswärme abgebenden Heizplatte gehalten werden.

## Beschreibung

Die Erfindung betrifft ein Verfahren zur Freisetzung von Wirkstoffen, insbesondere ätherischen Wirkstoffen, aus Heilpräparaten, vorzugsweise geschnittenen Kräutern bzw. Kräutermischungen, in einen abgegrenzten Raum zwecks Langzeit-Behandlung von Erkrankungen der Atemwege wenigstens eines im Raum befindlichen Patienten, sowie eine Vorrichtung zur Durchführung des Verfahrens.

Zur Behandlung von Erkrankungen der Atemwege ist es bekannt, aus Heilpräparaten freigesetzte Wirkstoffe zu inhalieren.

Die einfachste Methode zur Bereitstellung inhalierbarer Wirkstoffe besteht darin, eine Schale mit heißem Wasser zu benutzen, und dem Wasser eine vorbestimmte Menge eines jeweils gewünschten Heilpräparates zuzusetzen. Die aus dem heißen Wasser aufsteigenden Dämpfe sind mit freigesetzten Wirkstoffen angereichert und können inhaliert werden.

Neben dieser sogenannten Warminhalation ist es auch bekannt, Inhaliergeräte zu benutzen, die z.B. flüssige Heilpräparate zu inhalierbaren Nebeln zerstäuben.

Während die Aufbereitung mit heißem Wasser den Nachteil hat, daß z.B. aus Heitkräutern lediglich nur ein Teil der darin enthaltenen Wirkstoffe freigesetzt werden, hat das Zerstäuben den Nachteil, daß dafür flüssige Heilpräparate zur Verfügung stehen müssen.

Darüber hinaus haben beide Methoden den Nachteil gemeinsam, daß die Freisetzung von Heildämpfen und -nebeln auf einen relativ kurzen Zeitraum beschränkt ist, vor allem bei der einfachen Warminhalation mit heißem Wasser tritt auch noch der Nachteil auf, daß die Konzentration der Wirkstoffe und die Temperatur am Anfang für den Patienten unangenehm hoch sein kann und dann mit zunehmender Abkühlung rasch abnimmt. Der Behandlungsvorgang bedarf folglich oftmaliger Wiederholung, zumal effektiv heilende Wirkstoffe lediglich einen mehr oder weniger geringen Anteil an der Gesamtmenge, z.B. aus aufgebrühten Kräutern insgesamt freisetzbaren ätherischen Stoffen haben. Dies ist insbesondere bei Kamille der Fall, bei der bedeutsame Wirkstoffe nicht oder nur zum Teil durch die Aufbrühmethode mit heißem Wasser freigesetzt werden. Ein wesentlicher Teil der Wirkstoffe bleibt unaufgeschlossen und wird nicht nutzbar freigesetzt.

Der Erfindung liegt die Aufgabe zugrunde, ein Verfahren bereitzustellen, mit dem sich wesentliche und bedeutsame Wirkstoffe insbesondere aus Kräutern derart freisetzen lassen, daß eine Langzeitbehandlung der Erkrankungen der Atemwege durch Inhalation der Wirkstoffe möglich ist, sowie Vorrichtungen bereitzustellen, die bei minimalem apparativem Aufwand eine Inhalation aus Kräutern und Kräutermischungen freigesetzte Wirkstoffe mit hohem Wirkungsgrad auf einfachste Art und Weise ermöglichen.

Diese Aufgabe ist erfindungsgemäß dadurch gelöst, daß in ein topfartiges Gefäß gefülltes Wasser mittels einer regelbaren Heizeinrichtung auf eine vorbestimmte, unterhalb des Siedepunktes liegende Temperatur erwärmt und von der Heizeinrichtung über einen vorbestimmten Zeitraum auf der vorbestimmten Temperatur gehalten wird, daß in ein zum Raum wenigstens teilweise offenes Kräuter-Reservoir Kräuter bzw. Kräutermischung eingegeben wird und daß das gefüllte Reservoir in das Wasserbad des topfartigen Gefäßes eingesetzt wird, derart, daß die im Reservoir enthaltenen Kräuter bzw. Kräutermischung ausschließlich der Wärmeeinwirkung des Wassers ausgesetzt und dabei ein direkter Kontakt zwischen dem Wasser und den Kräutern bzw. der Kräutermischung vermieden wird.

Die Erwärmung der Heilpräparate erfolgt praktisch in einem Wasserbad und ist mit Vorteil besonders schonend. Die Temperaturen sind niedrig, so daß es nicht zu nachteiligen Überhitzungen der Präparate kommen kann. Aus den Heilkräutern werden die Wirkstoffe langsam und über einen relativ langen Zeitraum bei ständiger milder Wärmenachfuhr freigesetzt. Durch die schonende und trockene Erwärmung werden auch solche Wirkstoffe freigesetzt, die bei sonst üblichen Aufbrühmethoden unaufgeschlossen bleiben. Dies ist insbesondere bei Kamille der Fall.

Gleichzeitig verdunstet auch das erwärmte Wasser verhältnismäßig langsam, wobei mit vorteilhafter Langzeitwirkung die relative Luftfeuchtigkeit im Behandlungsraum erhöht wird, was der gewünschten Heilbehandlung der Atemwege förderlich ist.

Ein Patient kann z.B. während seiner Nachtruhe im Schlafraum mit jedem Atemzug die in der Umgebungsluft im Raum befindlichen Wirkstoffe einatmen. Dadurch erfolgt eine Langzeitbehandlung der Atemwege und sogar der Nebenhöhlen im Nasen-Rachenraum.

Durch die langsame, schonende Erwärmung der Heilpräparate, insbesondere von Kräutern, ohne daß diese in Kontakt mit dem lediglich als Wärmeüberträger dienenden Wasser kommen, werden, wie bereits gesagt, aus den Kräutern auch solche Wirkstoffe freigesetzt und somit für Heilbehandlungen nutzbar gemacht, die bei einem sonst üblichen Aufbrühen, wenn überhaupt eine Freisetzung erfolgt, allenfalls im Brühwasser enthalten sind und somit nicht als nutzbare Dämpfe zur Verfügung stehen.

Es ist bekannt, daß die meisten wertvollen Wirkstoffe eines Kräuteraufgusses mit dem Brühwasser ungenutzt entsorgt werden. Dieser Nachteil wird erfindungsgemäß vermieden, indem die Heilpräparate, insbesondere Kräuter, besonders schonend und trocken erwärmt werden, wodurch die wertvollen Substanzen aufgeschlossen und in für eine Langzeitbehandlung geeigneter Weise freigesetzt werden.

Selbstverständlich können auch Salben, Tinkturen und sonstige Formen von Heilpräparaten auf die erfindungsgemäße Art und Weise erwärmt, aufgeschlossen und für eine Langzeitbehandlung genutzt werden.

Das erfindungsgemäße Verfahren zeichnet sich mit Vorteil desweiteren dadurch aus, daß zu seiner Durchführung keine aufwendige Apparatur notwendig ist. Das benötigte Gefäß mit Heizeinrichtung kann z.B. ein an sich bekannter Haushalts-Wasserkocher, ein sogenannter 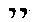 Blitzkocher", mit einem ein Fassungsvermögen von beispielsweise nicht mehr als einem Liter aufweisenden Topf mit integrierter elektrischer, thermostatgeregelter Heizeinrichtung sein.

Derartige Geräte sind in den meisten Haushalten vorhanden bzw. werden preiswert vom einschlägigen Fachhandel angeboten.

Eine besonders geeignete Vorrichtung, die auch langsam und schonend erwärmt bzw. das eingefüllte Wasser während einer langen Zeit auf einer vorbestimmten Temperatur halten kann, ist ein an sich bekannter Erwärmer für Babynahung.

Das Reservoir, das Heilpräparate enthält, die erwärmt werden sollen, kann ein einfaches Gefäß, z.B. eine Flasche oder ein Glas sein, das in das Gefäß mit Heizeinrichtung gestellt wird. Vorzugsweise ist das Reservoir eine auf die Oberfläche des im Gefäß eingegebenen Wassers setzbare Schwimmwanne. In die Schwimmwanne können die Heilpräparate gefüllt werden. Die Schwimmwanne ist selbstverständlich so bemessen, daß selbst bei vollständiger Füllung mit Heilpräparaten noch genügend Auftrieb verbleibt, um die gefüllte Schwimmwanne schwimmfähig zu halten. Die oben offene Wannenform gewährleistet die Freisetzung der Wirkstoffe aus der Wannenfüllung, wobei die Wärme des Wassers auf die darin schwimmende Wanne und deren Inhalt übertragen wird. Selbstverständlich können auch andere Gefäße mit entsprechenden Auftriebselementen, die eine Schwimmfähigkeit gewährleisten, verwendet werden.

Nach einer vorteilhaften Weiterbildung umfaßt die Schwimmwanne einen Napf mit einem äußeren, scheibenförmigen bzw. flanschartigen Schwimmkragen. Der Schwimmkragen sorgt für eine stabile Schwimmlage und hält die schwimmende Schwimmwanne im Zentrum des topfartigen Gefäßes. Die Zentrierung ist insbesondere vorteilhaft, weil der Durchmesser des Schwimmkragens geringer ist als der Innendurchmesser des topfartigen Gefäßes, in welchem die Schwimmwanne schwimmt, damit noch eine freie Wasseroberfläche für eine langsame, dem Raumklima förderliche Wasserverdunstung verbleibt.

Weil die Schwimmwanne zusammen mit ihrem Schwimmkragen die freie Wasseroberfläche im Gefäß jedoch verkleinert, könnte die an und für sich für die Herstellung eines verbesserten Raumklimas gewünschte Wasserverdunstung vermindert sein. Um solche Verminderung der Wasserverdunstung zu vermeiden, kann der Schwimmkragen mit Durchbrüchen versehen werden oder sogar nur aus einigen wenigen, radial von der Schwimmwanne bzw. dem Napf der Schwimmwanne abstehenden Speichen, Stegen, Rippen oder dergl. bestehen.

Die Wirkungsweise der Vorrichtung, insbesondere ihre Handhabung, wird nach einer anderen Weiterbildung noch dadurch verbessert, daß ein in das Gefäß einsetzbarer Einsatz vorgesehen ist, dessen Außenwandung parallel zur Innenwand des Gefäßes verläuft und der einen umlaufenden Außenkragen zur Auflage auf den Öffnungsrand des Gefäßes hat. Zweckmäßigerweise ist der Einsatz bei einem zylindrischen Gefäß als Hülse bzw. Hohlzylinder ausgebildet, dessen Innendurchmesser größer als der Außendurchmesser des Schwimmkragens der Schwimmwanne ist. Unterhalb des Außenkragens befindliche Wandabschnitte des Einsatzes sind somit unter die Oberfläche des im Gefäß befindlichen Wassers abgetaucht, wenn der Einsatz mit seinem Außenkragen auf dem Öffnungsrand des Gefäßes liegt. Die Schwimmwanne schwimmt innerhalb des Einsatzes, dessen Außenwandung die Innenwand des Gefäßes abdeckt. Verdunstet das Wasser und fällt der Wasserspiegel innerhalb des Einsatzes während der Dauer der Anwendung des erfindungsgemäßen Verfahrens, bewirkt diese Abdeckung, daß keinerlei freigesetzte Wirkstoffe mit der Innenwand des topfartigen Gefäßes in Kontakt kommen. Dadurch ist sichergestellt, daß keine unkontrollierbare, nicht vorhersehbare chemische Reaktionen zwischen dem Material der Wand des topfartigen Gefäßes und den freigesetzten Wirkstoffen erfolgen kann.

Optimal ist eine Anpassung der Länge des Einsatzes an die Tiefe des topfartigen Gefäßes, derart, daß der untere Rand des Einsatzes nahezu bis zum Grund des Gefäßes reicht. Dies könnte z.B. durch einfaches Abschneiden oder Ablängen eines hülsenförmigen Einsatzes aus weitgehend chemisch resistentem Material zwecks Anpassung an die Tiefe des vorhandenen Gefäßes erfolgen.

Einfacher ist es jedoch, eine Verschiebemöglichkeit des Außenkragens vorzusehen. Der Außenkragen könnte z.B. an einer den Einsatz umfassenden Schiebehülse angeordnet sein.

Der Einsatz kann auch derart gestaltet sein, daß in ein aus dem Gefäß vorstehendes Einsatz-Oberteil wenigstens ein Siebboden einsetzbar ist. In den Siebboden können zusätzlich zum Inhalt der Schwimmwanne noch solche Heilpräparate eingegeben werden, die weitere Wirkstoffe an die von unten durch den Siebboden ziehenden Dämpfe abgeben. So könnte z.B. bei eingesetztem Siebboden auch auf die Schwimmwanne verzichtet. Denkbar ist es desweiteren, in die Schwimmwanne eine Salbe einzugeben und auf den Siebboden geeignete Kräuter. Desgleichen kann die Schwimmwanne eine erste Kräutermischung aufnehmen und dann auf den Siebboden eine zweite Kräutermischung gegeben werden.

Zweckmäßigerweise sind der Einsatz, die Schwimmwanne und der Siebboden aus geeignetem, weitgehend chemisch resistentem Kunststoff hergestellt. Damit die Schwimmwanne dann, wenn z.B. der Wasserinhalt des Gefäßes weitgehend verdunstet ist, nicht auf dem erhitzten Boden des Gefäßes aufsetzt und beschädigt wird, können an der Innenwand des Einsatzes in vorbestimmter Tiefe nach innen vorstehende Auflageschultern in Form von Wulsten, Nippeln oder dergl. Vorsprüngen vorgesehen sein, auf denen der Schwimmkragen der Schwimmwanne aufsetzen kann, bevor die Schwimmwanne mit dem Gefäßboden in Kontakt kommt.

Der als Schwimmwanne dienende Napf kann auch seinen Innenraum abteilende Schottwände aufweisen. Dadurch ist es mit Vorteil möglich, die Menge der aufzudunstenden Kräuter bzw. Kräutermischungen entweder zu variieren, indem lediglich eines oder mehrere der zur Verfügung stehenden Abteile des Innenraums der Schwimmwanne gefüllt werden oder auch in verschiedene Abteile unterschiedliche Kräuter oder Kräutermischungen eingebbar sind, die dann gleichzeitig erfindungsgemäß aufgedunstet werden.

Damit eine lediglich zum Teil und damit einseitig beladene Schwimmwanne eine horizontale Schwimmlage beibehält ist nach einer Weiterbildung vorgesehen, daß eine in das Gefäß vom oberen Öffnungsrand gegen den Gefäßboden vorstehende Führung für die Schwimmwanne vorhanden ist, mittels der die Schwimmwanne dem fallenden Wasserstand im Gefäß nachführbar ist.

Eine konstruktiv einfache und somit auch vorteilhaft unkompliziert handhabbare Ausgestaltung sieht vor, daß die Führung einen am oberen Öffnungsrand des Gefäßes ansetzbaren Jochbügel umfaßt, der ein Halteorgan für eine die Schwimmwanne führende Stange aufweist.

Das Halteorgan kann z.B. eine Schraubklemme sein, durch welche die als Führung dienende Stange verläuft, mit dem Vorteil, daß die Schraubklemme eine Verstellung und Justierung der Länge desjenigen Abschnitts der Stange erlaubt, welcher in das Gefäß eintaucht und der folglich auch die Länge des Führungsweges vorgeben kann.

Die geführte Bewegung der Schwimmwanne entlang der Stange wird mit Vorteil dadurch erreicht, daß die Schwimmwanne eine auf ihrem Boden über einer Bodenöffnung stehende Hülse aufweist, durch die die als Führung dienende Stange verläuft. Vorzugsweise sind Bodenöffnung und Hülse bei einer Runden Schwimmwanne in deren Zentrum angeordnet.

Die Stange kann somit den Boden durchdringen und dennoch tritt kein Wasser in die schwimmende Schwimmwanne ein.

Der Führungsweg, bzw. die Senkbewegung der geführten Schwimmwanne bei aufgrund einer Verdunstung abnehmendem Wasserstand im Gefäß läßt sich mit Vorteil dadurch begrenzen, daß am aus dem Boden der Schwimmwanne gegen den Boden des Gefäßes vorstehenden Ende der als Führung dienende Stange ein dem Fallweg der Schwimmwanne begrenzender Anschlag angeordnet ist. Der Anschlag kann eine einfache Verdickung am freien Ende der Stange sein, die nicht durch die Bodenöffnung paßt. Mittels der Klemmschraube kann, wenn das Sinken des Wasserspiegels zeitlich erfaßt wird, was z.B. empirisch erfolgen kann, auch eine Art Betriebsdauer der Aufdunstung eingestellt werden, weil der Anschlag bewirken kann, daß die Schwimmwanne nach einer vorbestimmten Verdunstungszeit des Wassers aus dem Wasser gehoben wird, womit dann das erfindungsgemäße Verfahren abgebrochen wäre.

Eine andere Ausführungsmöglichkeit für eine Vorrichtung, mit die Aufgabe vorrichtungsmäßig gelöst wird und für die auch selbständiger Schutz beansprucht wird, zeichnet sich aus durch eine Wärmequelle mit als Heizplatte ausgebildeter waagerechter Stellfläche, durch ein auf die Stellfläche stellbares Ständerteil und durch ein vom Ständerteil derart gehaltenes Kräuterreservoir, daß sich dessen Boden in jeweils vorbestimmbaren Abständen über der Stellfläche befindet.

Die Heizplatte als Wärmequelle erwärmt das im darauf abgestellten Ständerteil gehaltene Kräuterreservoir und damit die darin enthaltenen Kräuterportionen besonders schonend. Eine Überhitzung und dadurch verursachte Verbrennungen findet nicht statt, da das Ständerteil den Boden des Kräuterreservoirs in einem vorteilhaften Abstand über der Stellfläche hält, so daß das Kräuterreservoir keinen direkten Kontakt mit der als Wärmequelle dienenden Heizplatte hat. Es erwärmt sich lediglich die über der Heizplatte stehende Luft, die gleichmäßig aufsteigt und dabei Boden und Seitenwände des Kräuterreservoirs gleichmäßig umströmt und schonend erwärmt.

Die auf die Kräuter im Kräuterreservoir einwirkende milde Erwärmung erfolgt dabei mit relativ niedriger Temperatur zwischen 40°C und 80°C. Die Erwärmung kann mit Vorteil relativ lange Zeit, z.B mehrere Stunden aufrecht erhalten werden. Die erwärmten, bzw. warmgehaltenen Kräuter im Kräuterreservoir dunsten während der gesamten Betriebszeit der Wärmequelle dabei die in ihnen enthaltenen ätherischen Wirkstoffe aus, die in die Umgebung, z.B. einen geschlossenen Raum, in dem die Vorrichtung betrieben wird, freigesetzt werden. Während sich eine Person in dem Raum aufhält, kann sie die in der Raumluft enthaltenen, aus den Kräutern freigesetzten Wirkstoffe mit der Atemluft aufnehmen.

Die relativ langsame, langandauernde und damit schonende und milde Wärmezufuhr hat auch den Vorteil, daß die im Kräuterreservoir befindliche Kräuteranhäufung gleichmäßig durchwärmt wird, so daß die Entstehung von kälteren Bereichen, in denen ein Aufschluß der Kräuter nicht stattfindet, ausgeschlossen ist.

Das Ständerteil ist ein konstruktiv einfaches Bauteil, in welches das Reservoir z.B. eingesetzt werden kann. Vorzugsweise ist das Ständerteil als Hohlzylinder ausgebildet, der, auf der Stellfläche der Heizplatte aufgestellt, nur relativ kleine Kontaktflächen mit der Heizplatte hat. Dadurch erfolgt nur eine unbedeutende Wärmeeinleitung in das Ständerteil, wobei die eingeleitete Wärme über die im Verhältnis zur Kontaktfläche mit der Heizplatte relativ große Oberfläche der Wandungen des Hohlzylinders auch wieder in hohem Maße abgegeben wird. Das Ständerteil selbst wird somit nicht auf so hohe Temperaturen aufgeheizt, daß es wiederum über Kontakte und Warmebrucken das im Ständerteil aufgenommene Kräuterreservoir übermäßig erwärmen würde. Gleichzeitig durchströmt warme Luft den schornsteinänhlich wirkenden Hohlzylinder und überträgt seine Wärme bei der Umströmung des Kräuterreservoirs an dessen Wandungen.

Damit das Kräuterreservoir durch einfache, die Handhabung der Vorrichtung erleichternde Maßnahmen in das Ständerteil eingesetzt werden kann, weist der als Ständerteil dienende Hohlzylinder an einem Ende eine nach Innen gerichtete, seinen freien Innenquerschnitt verengende Einziehung auf.

Die Einziehung hat den Vorteil, daß sie eine Auflageschulter für ein Kräuterreservoir bildet. Darüber hinaus bewirkt die einseitige Anordnung einer solchen Einengung, daß durch einfaches Umdrehen und entsprechendes Aufstellen des Ständerteils auf die Stellfläche der Heizplatte, entweder mit dem die Einengung aufweisenden Ende oder mit dem einengungslosen Ende, daß der Boden des Kräuterreservoirs einmal in einem größeren Abstand über der Stellfläche der Heizplatte steht, wenn das Kräuterreservoir auf der Einengung liegt und zum anderen zur Stellfläche nur einen geringeren Abstand aufweist, wenn das Kräuterreservoir auf dem einengungslosen Ende des hohzylinderförmigen Ständerteils ruht.

Die Innenwand des hohlzylinderförmigen Ständerteils weist als Distanzhalter für ein Kräuterreservoir wirkende Vorsprünge auf. Abgesehen von der das Kräuterreservoir zentrierenden Wirkung fördern die Distanzhalter auch die Luftdurchströmung des Ständerteils, zumal dann, wenn sie mit Vorteil als nach innen vorstehende lotrechte Leisten ausgebildet sind, die z.B. angesetzt oder angeformt sein können.

Jedes Kräuterreservoir ist als einseitig offenes Gefäß ausgebildet. Für den erfindungsgemäßen Einsatz ist ein als runder Napfausgebildetes Kräuterreservoir besonders gut geeignet, der einen Außendurchmesser aufweist, welcher einem durch die Vorstehung der Distanzhalter des Ständerteils vorgegebenen Innendurchmesser des hohlzylinderförmigen Ständerteils entspricht.

Ein solches, als Napf ausgebildetes Kräuterreservoir kann in das zylinderförmige Ständerteil auf einfachste Art und Weise eingehängt werden, sobald das Ständerteil auf der Stellfläche der Heizplatte aufgestellt ist.

Die Einhängung wird dadurch erleichtert, daß das Kräuterreservoir am oberen Öffnungsrand radial vorstehende Halter aufweist.

Nach einer vorteilhaften Weiterbildung ist als Halter ein umlaufender Kragenflansch vorgesehen.

Mit besonderem Vorteil steht der Kragenflansch trichterartig geneigt zum Öffnungsrand des Kräuterreservoirs. Mit dem Kragenflansch kann das Kräuterreservoir auf den jeweils oberen Rändern des zylinderförmigen Ständerteils aufliegen, wobei der Boden des als Napf ausgebildeten Kräuterreservoirs in das Ständerteil abgetaucht ist.

Bei im Ständerteil somit hängenden Kräuterreservoir befindet sich sein Boden in dem durch die Höhe des zylinderförmigen Ständerteils bestimmten Abstand über der Stellfläche der Heizplatte.

Das Kräuterreservoir kann einen beliebig gestalteten Boden aufweisen. Zweckmäßigerweise hat das Kräuterreservoir einen ebenen Boden, welcher den Vorteil hat, daß er parallel zur Stellfläche der Heizplatte verläuft und somit gleichmäßiger aufgeheizt wird. Jeder Flächenbereich des Bodens des Kräuterreservoirs hat zur Stellfläche der Heizplatte den gleichen Abstand, was bei einem gewölbten Boden nicht der Fall wäre.

Auf eine Heilbehandlung mit aus Kräutern durch Wärmezufuhr freigesetzten ätherischen Wirkstoffen hat das Volumen eines Raumes, in dem die erfindungsgemäße Vorrichtung aufgestellt wird, nicht unerheblichen Einfluß. Um eine höhere Konzentration freigesetzter ätherischer Wirkstoffe zu erreichen müssen mit der Vorrichtung z.B. größere Mengen von Kräutern behandelbar sein. Auch für einen größeren Raum ist, um eine ausreichende Konzentration zu erreichen, eine größere Kräutermenge mit der Vorrichtung zu behandeln. Variationsmöglichkeiten ermöglicht die erfindungsgemäße Vorrichtung dadurch, daß in das Kräuterreservoir einstellbare weitere Kräuteraufnahmen vorgesehen sind. Das Kräuterreservoir ist so bemessen, daß es eine maximale mögliche große Kräutermenge aufnehmen kann. Durch in das Kräuterreservoir einstellbare weitere Kräuteraufnahmen läßt sich, je nach Durchmesser der Kräuteraufnahmen, das Volumen der darin aufgenommenen Kräuterportion vermindern.

Konstruktiv einfache und damit vorteilhaft gestaltete Kräuteraufnahmen sind so ausgebildet, daß jede Kräuteraufnahme ein Hohlzylinder ist, der mit seinem unteren Rand auf den Boden des Kräuterreservoirs in das Kräuterreservoir hineinstellbar ist. Kräuteraufnahmen mit unterschiedlichen Durchmessern und somit unterschiedlichen Aufnahmevolumina sind ohne weiteres bereitstellbar. So kann z.B. jeder Vorrichtung ein Satz unterschiedlicher Kräuteraufnahmen beigegeben werden, die auch so bemessen sein können, daß sie ineinander stapelbar sind. Jede Kräuteraufnahme ist bodenlos, da sie den Boden des Kräuterreservoirs mit nutzt.

Jede Kräuteraufnahme weist am oberen Rand einen Kragenflansch auf. Der Kragenflansch ist mit Vorteil trichterartig geneigt. Der Kragenflansch hat den Vorteil, daß er die Kräuteraufnahme im Kräuterreservoir zentriert, wobei die trichterartige Neigung des Kragenflansches das Einschütten von Kräutern in die Kräuteraufnahme erleichtert. Der Ringraum zwischen der Innenwand des Kräuterreservoirs und der Außenwand der Kräuteraufnahme wird durch den vorstehenden Kragenflansch mit Vorteil verschlossen.

Der Werkstoff für das Ständerteil, das Kräuterreservoir und die Kräuteraufnahme ist vorzugsweise Glas. Dieser Werkstoff ist in hygienischer Hinsicht vorteilhaft und die entsprechenden Bauteile lassen sich durch Preßformen leicht und kostengünstig herstellen.

Als Wärmequelle ist jede Einrichtung geeignet, die in der Lage ist, Wärmeenergie mit nicht allzu hohen Oberflächentemperaturen über einen langen Zeitraum abzugeben. Die Temperaturen sollen bei etwa 50 bis 70°C liegen, jedoch den Siedepunkt von Wasser nicht erreichen. Als Wärmequelle, die diese Eigenschaften bietet, ist eine elektrische Haushaltswärmeplatte, insbesondere ein elektrisches Tee-Stövchen besonders geeignet.

Ausführungsbeispiele der Erfindung, aus denen sich weitere erfinderische Merkmale ergeben, sind in der Zeichnung dargestellt.

Es zeigen:
- Fig. 1: eine Ansicht einer Vorrichtung zur Durchführung des Verfahrens nach Art einer Explosionsdarstellung,
- Fig. 2: eine Seitenansicht der Vorrichtung entsprechend Fig.1 in einem Schnitt,
- Fig.3: eine Seitenansicht einer anderen Ausführung der Vorrichtung mit einer Stell-Führung für die Schwimmwanne,
- Fig.4: eine Vorrichtung gemäß Fig.3 in einer um 90° gegenüber Fig.3 gedrehten Seitenansicht,
- Fig.5: eine schematische Seitenansicht einer weiteren Ausführungsmöglichkeit der Vorrichtung,
- Fig.6: eine Seitenansicht des Ständerteils der Vorrichtung gemäß Fig. 5 im Schnitt,
- Fig.7: die Draufsicht des Ständerteils gemäß Fig.6 und
- Fig.8: eine schematische Seitenansicht entsprechend Fig.5, jedoch mit gegenüber Fig.5 um 180° gedreht stehendem Ständerteil.

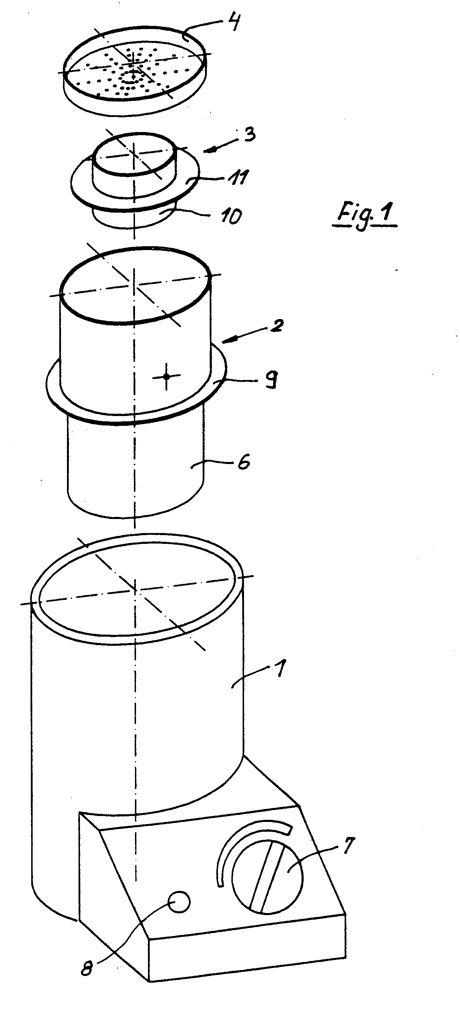

In Fig.1 ist eine Explosionsdarstellung einer Vorrichtung zur Freisetzung von Wirkstoffen aus Heilpräparaten gezeichnet. Ein topfartiges Gefäß 1, bei diesem Ausführungsbeispiel ein handelsübliches Gerät zur Erwärmung und Warmhaltung von Babynahrung, ist mit einer nicht weiter dargestellten elektrischen Heizeinrichtung ausgerüstet, die hier durch den Regelungsknopf 7 in Verbindung mit der Kontrollampe 8 lediglich angedeutet ist. In das topfartige Gefäß 1, das oben offen ist, kann Wasser eingefüllt werden, das bei entsprechender Einstellung des Regelungsknopfes 7 auf eine vorbestimmte Temperatur erwärmt und warmgehalten wird.

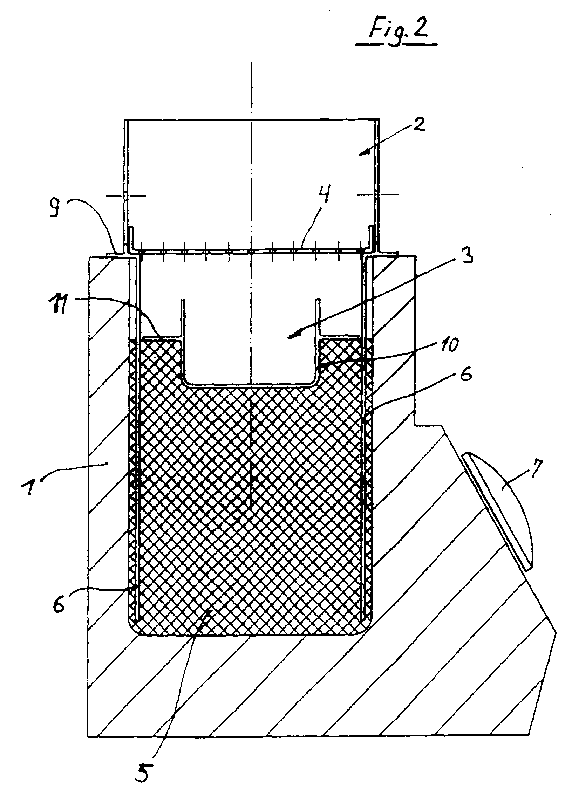

In das topfartige Gefäß 1 ist ein dazu passender, als Hülse bzw. Hohlzylinder ausgebildeter Einsatz 2 einsteckbar. Der Einsatz hat einen umlaufenden Außenkragen 9, der auf dem oberen Öffnungsrand des topfartigen Gefäßes 1 aufliegen kann, so daß sich die Außenwandung 6 des Einsatzes 2 in das Gefaß 1 hinein erstreckt und unter die Oberfläche des eingefüllten Wassers abgetaucht ist ( Fig. 2).

In den Einsatz 2 ist ein Reservoir 3 für Heilpräparate einsetzbar. Das Reservoir 3 ist hier als oben offene Schwimmwanne 10 ausgebildet, die napfförmig ist und einen äußeren, flanschförmig angeordneten Schwimmkragen 11 aufweist. Der Außendurchmesser des Schwimmkragens 11 ist geringer als der Innendurchmesser des Einsatzes 2.

In die napfförmige Schwimmwanne ist eine Portion Heilkräuter, z.B. Kamille oder eine Salbe bzw. Öl oder dergl. Heilpräparat eingebbar.

Ist der Einsatz 2 in das mit Wasser gefüllte Gefäß 1 eingesetzt, kann die befrachtete Schwimmwanne 10 von oben in den Einsatz 2 eingegeben werden, so daß sie auf der im Einsatz 2 stehenden Oberfläche des Wassers schwimmt. Wird das Wasser erwärmt, wird die Wärme des Wassers auch durch die Wand der Schwimmwanne 10 geleitet und der Inhalt der Schwimmwanne äußerst schonend erwärmt. Durch die schonende, trockene Erwärmung werden Wirkstoffe aus dem in der Schwimmwanne 10 befindlichen Heilpräparat freigesetzt, die an die normale Raumluft im Raum, in dem das Gefäß 1 aufgestellt ist, abgegeben werden.

In den oberen Bereich des Einsatzes 2, der sich über der Wasseroberfläche befindet bzw. der aus dem Gefäß 1 über dessen oberen Öffnungsrand vorsteht, kann auch noch zusätzlich ein Siebboden 4 eingesetzt werden, auf den z.B. eine zweite Kräutermischung gelegt werden kann. Der Siebboden 4 kann selbstverständlich auch das Reservoir 3 ersetzen, das heißt, für sich allein als Reservoir genutzt werden.

Fig.2 zeigt eine Seitenansicht einer betriebsbereit zusammengesetzten Vorrichtung im Schnitt. Das eingefüllte Wasser 5 ist durch Kreuzschraffur angedeutet. Gleiche Bauteile sind mit gleichen Bezugszahlen wie in Fig. 1 bezeichnet.

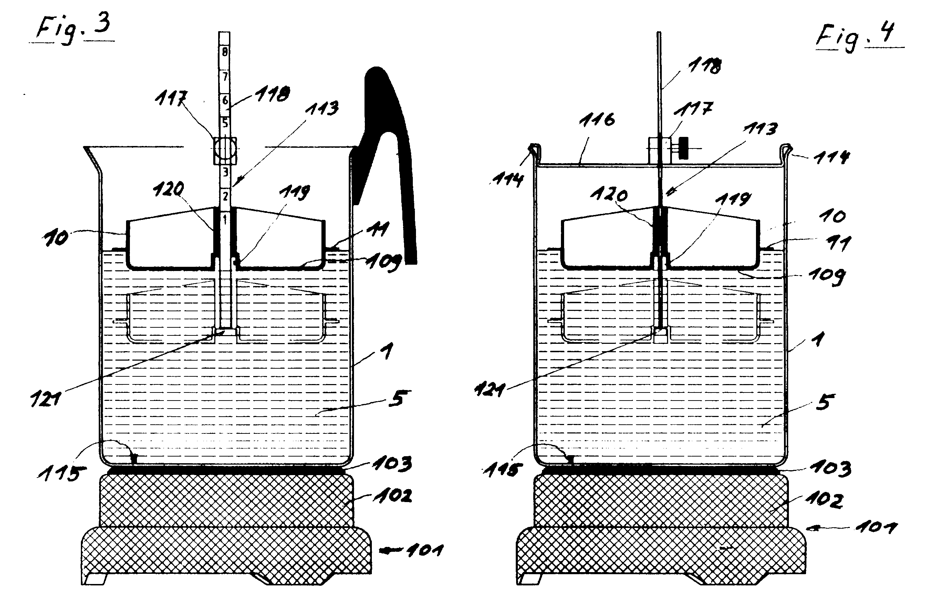

Fig.3 zeigt in einer Seitenansicht eine Vorrichtung bei der ein als Schwimmwanne 10 dienender Napf seinen Innenraum abteilende Schottwände aufweist. Ein kreisförmiger Napf kann z.B. mittels radial verlaufender Schottwände in Viertel-Segment-Abteile geteilt sein. Es ist eine in das Gefäß 1 vom oberen Öffnungsrand 114 lotrecht gegen den Gefäßboden 115 vorstehende Führung 113 vorgesehen, die hier als in das Gefäß 1 zentral ragende Stange 118 ausgebildet ist. Mittels der Führung 113 ist die Schwimmlage einer teilbeladenen Schwimmwanne 10 stabilisiert, während sie dem aufgrund laufender Verdunstung fallenden Wasserstand im Gefäß 1 nachgeführt wird.

Die Führung 113 umfaßt einen am oberen Öffnungsrand 114 des Gefäßes 1 ansetzbaren Jochbügel 116 aus Kunststoff oder Metalldraht, der ein hier als Klemmschraube ausgebildetes Halteorgan 117 für die als Stange 118 ausgebildete Führung 113 aufweist. Die Schwimmwanne 10 hat eine auf ihrem Boden 109 über einer Bodenöffnung 119 stehende Hülse 120, durch welche die führende Stange 118 verläuft. An dem aus dem Boden 109 herausgeführten freien Ende der Stange 118 ist ein den Fallweg der Schwimmwanne 10 begrenzendes Anschlagorgan 121, hier eine einfache Verdickung, angeordnet.

Zur Erleichterung der Einstellung eines jeweils gewünschten Fallweges der Schwimmwanne 10 weist die Stange 118 eine Skaleneinteilung auf wie sie hier angedeutet ist.

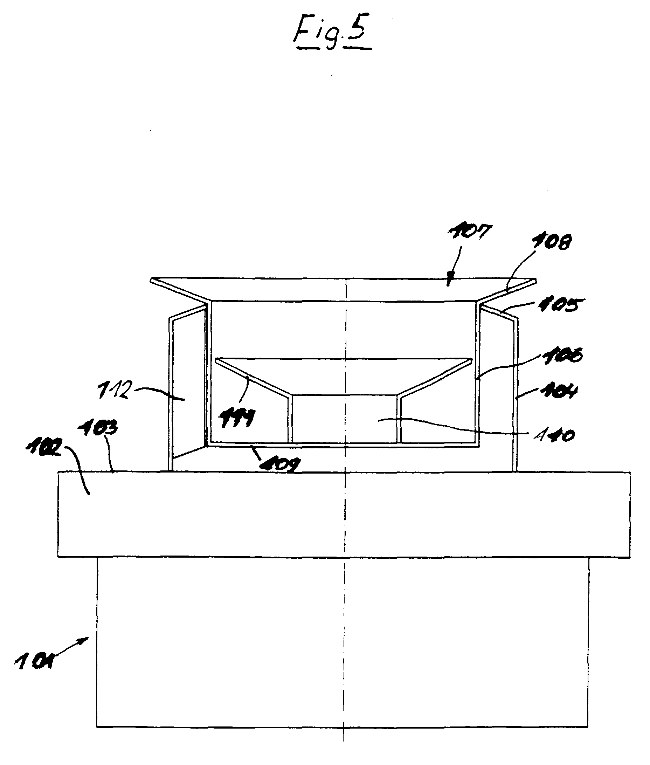

Fig.4 zeigt die Vorrichtung gemäß Fig.3 in einer gegenüber Fig.3 um 180° gedrehten Seitenansicht. Gleiche Bauteile sind mit gleichen Bezugszahlen wie in Fig.3 bezeichnet.

In Fig.5 ist die weitere Ausgestaltungsmöglichkeit für eine Vorrichtung in einer schematischen Seitenansicht dargestellt. Ein handelsübliches, elektrisches Tee-Stövchen dient als Wärmequelle 101. Es weist eine Heizplatte 102 auf, die eine obere waagerechte Stellfläche 103 hat. Auf der Stellfläche 103 steht ein als Hohlzylinder ausgebildetes Ständerteil 104, dessen unteres Ende eine nach Innen gerichtete, seinen freien Innenquerschnitt verengende Einziehung 105 aufweist. In dem Ständerteil 104 ist ein napfförmiges Kräuterreservoir 106 gehalten. Das Kräuterreservoir 106 weist an seinem oberen Öffnungsrand radial vorstehende Halter 107 auf, wobei als Halter 107 bei diesem Ausführungsbeispiel ein angeformter Kragenflansch 108 vorgesehen ist. Der Kragenflansch 108 steht trichterartig geneigt zum Öffnungsrand des Kräuterreservoirs 106, so daß das Kräuterreservoir 106 mit dem Kragenflansch 108 auf dem oberen Rand des Ständerteils 104 aufliegt, sobald es in das Ständerteil 104 eingehängt ist. Standerteil 104 und Kräuterreservoir 106 sind dabei so dimensioniert, daß der Boden 109 des napfförmig ausgebildeten Kräuterreservoirs 106 sich in einem vorbestimmten Abstand über der Stellfläche 103 der Heizplatte 102 der Wärmequelle 101 befindet.

Bei in Betrieb gesetzter Wärmequelle wird Wärme von der Heizplatte 102 aufsteigen und das im Ständerteil 104 hängende Kräuterreservoir 106 von Außen erwärmen.

In das Kräuterreservoir 106 werden feingeschnittene getrocknete Kräuter in loser Schüttung eingegeben. Durch die milde Erwärmung werden in den Kräutern enthaltene ätherische Wirkstoffe freigesetzt und in die Umgebung ausgedunstet.

Soll eine kleinere Kräuterportion verwendet werden, besteht die hier angedeutete Möglichkeit, in das Kräuterreservoir 106 eine Kräuteraufnahme 110 einzustellen. Jede Kräuteraufnahme ist wiederum als oben und unten offener Hohlzylinder ausgebildet, der mit seinem unteren Rand auf dem Boden 109 des Kräuterreservoirs 106 steht. Jede Kräuteraufnahme weist ebenfalls am oberen Rand einen nach Außen vorstehenden trichterartig geneigten Kragenflansch 111 auf, welcher einerseits die Krauteraufnahme in dem Kräuterreservoir 106 zentriert und andererseits das Einschütten feingeschnittener Kräuter erleichtert. Kräuteraufnahmen mit jeweils unterschiedlichen zylindrischen Durchmessern des unteren Abschnitts ergeben unterschiedlich große Füllräume zur Aufnahme eingeschütteter Kräuter. Einer Vorrichtung kann ein Satz von Kräuteraufnahmen 110 mit jeweils unterschiedlichen Durchmessern beigegeben werden.

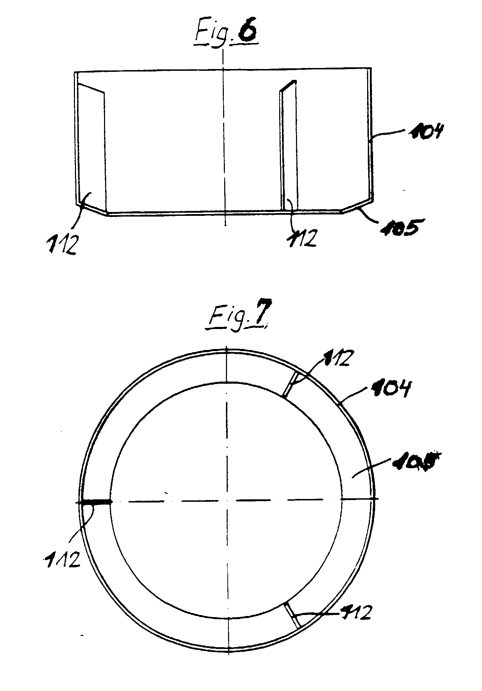

Fig.6 zeigt eine Seitenansicht des Ständerteils 104 im Schnitt.

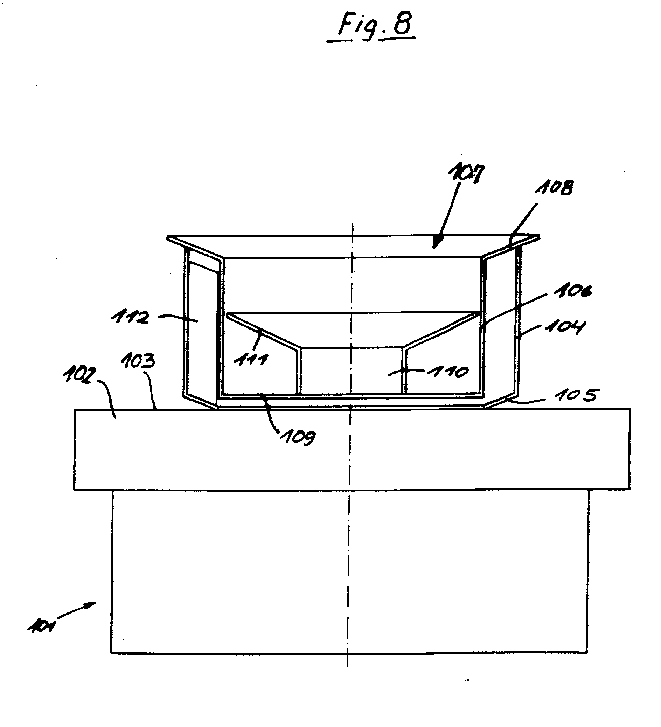

Fig.7 zeigt die Draufsicht des Ständerteils 104 gemäß Fig.6.

Die Fig.6 und 7 lassen erkennen, daß das Ständerteil 104 als Hohlzylinder ausgebildet ist. Der Hohlzylinder ist unten mit der nach Innen gerichteten, seinen freien Querschnitt verengenden Einziehung 105 versehen. Die Innenwand des als Hohlzylinder ausgebildeten Ständerteils 104 weist als Distanzhalter wirkende Vorsprünge auf wobei jeder Vorsprung bei diesem Ausführungsbeispiel als radial nach Innen vorstehende Leiste 112 ausgebildet ist.

Fig.7 läßt in Verbindung mit Fig.5 erkennen, daß die als Distanzhalter wirkenden Leisten 112 auch eine Zentrierung des in das Ständerteil 104 eingehängten Kräuterreservoirs 106 besorgen.

Fig.8 zeigt wiederum die Vorrichtung in einer Seitenansicht entsprechend Fig.5, wobei jedoch das Ständerteil 104 auf der Stellfläche 103 der Heizplatte 102 um 180° gedreht steht. Gleiche Bauteile sind mit gleichen Bezugszahlen bezeichnet. Fig.8 verdeutlicht, daß sich bei um 180° gedrehtem Ständerteil 104 der Boden 109 des Kräuterreservoirs 106 in einem größeren Abstand zur Stellfläche 103 der Heizplatte 102 befindet, so daß die Wärmezuleitung zum Kräuterreservoir 106 von der Heizplatte 102 der Wärmequelle 101 zum Kräuterreservoir vermindert ist. Dies kann bei bestimmten Kräutern von Vorteil sein. Wenn Kräuter aufgedunstet werden sollen, die zur Freisetzung ihrer ätherischen Wirkstoffe höhere Temperaturen benötigen, wird das Ständerteil 104 so verwendet aufgestellt, wie es in Fig.5 gezeigt ist. Bei Kräutern, die niedrigere Wärmezuführen erfordern, wird das Ständerteil gemäß Fig.8 aufgestellt.

## Patentansprüche

1. Verfahren zur Freisetzung von Wirkstoffen aus geschnittenen Kräutern bzw. Kräutermischungen in einen abgegrenzten Raum zwecks Behandlung von Erkrankungen der Atemwege eines im Raum befindlichen Patienten,
**dadurch gekennzeichnet,**
daß in ein topfartiges Gefäß (1) gefülltes Wasser (5) mittels einer regelbaren Heizeinrichtung auf eine vorbestimmte, unterhalb des Siedepunktes liegende Temperatur erwärmt und von der Heizeinrichtung über einen vorbestimmten Zeitraum auf der vorbestimmten Temperatur gehalten wird, daß in ein zum Raum wenigstens teilweise offenes Kräuter-Reservoir (3) Kräuter bzw. Kräutermischung eingegeben wird und daß das gefüllte Reservoir (3) in das Wasserbad des topfartigen Gefäßes (1) eingesetzt wird, derart, daß die im Reservoir (3) enthaltenen Kräuter bzw. Kräutermischung ausschließlich der Wärmeeinwirkung des Wassers (5) ausgesetzt und dabei ein direkter Kontakt zwischen dem Wasser (5) und den Kräutern bzw. der Kräutermischung vermieden wird.

2. Vorrichtung zur Durchführung des Verfahrens nach Anspruch 1, dadurch gekennzeichnet, daß das Gefäß (1) mit Heizeinrichtung ein an sich bekannter Haushalts-Wasserkocher mit einem ein Fassungsvermögen von nicht mehr als einem Liter aufweisenden Topf mit integrierter thermostatge-regelter Heizeinrichtung ist.

3. Vorrichtung zur Durchführung nach Anspruch 2, dadurch gekennzeichnet, daß das Reservoir (3) eine auf die Oberfläche des in das Gefäß (1) eingegebenen Wassers (5) setzbare Schwimmwanne (10) ist.

4. Vorrichtung nach Anspruch 3, dadurch gekennzeichnet, daß die Schwimmwanne (10) einen Napf mit äußerem, scheibenringförmigen Schwimmkragen (11) umfaßt, wobei der Durchmesser des Schwimmkragens (11) geringer als der Innendurchmesser des Gefäßes (1) ist.

5. Vorrichtung nach Anspruch 4, dadurch gekennzeichnet, daß der Schwimmkragen (11) Durchbrechungen aufweist.

6. Vorrichtung nach einem der Ansprüche 2 bis 5, gekennzeichnet durch einen in das Gefäß einsetzbaren Einsatz (2), dessen Außenwand (6) parallel zur Innenwand des Gefäßes (1) verläuft und der einen umlaufenden Außenkragen (9) zur Auflage auf dem Öffnungsrand des Gefäßes (1) hat.

7. Vorrichtung nach Anspruch 6, dadurch gekennzeichnet, daß der Einsatz (2) bei einem zylindrischen Gefäß (1) als hulsenförmiger Hohlzylinder ausgebildet ist, dessen Innendurchmesser größer als der Außendurchmesser des Schwimmkragens (11) der Schwimmwanne (10) ist.

8. Vorrichtung nach einem der Ansprüche 6 und 7, dadurch gekennzeichnet, daß der Einsatz (2) ein aus dem Gefäß (1) vorstehendes Oberteil aufweist, das als Aufnahme für wenigstens einen darin einsetzbaren Siebboden (4) ausgebildet ist.

9. Vorrichtung nach Anspruch 4, dadurch gekennzeichnet, daß der als Schwimmwanne (10) dienende Napf seinen Innenraum abteilende Schottwände aufweist.

10. Vorrichtung nach einem der Ansprüche 2 bis 9, dadurch gekennzeichnet, daß eine in das Gefäß (1) vom oberen Öffnungsrand (114) gegen den Gefäßboden (115) vorstehende Führung (113) für die Schwimmwanne (10) vorgesehen ist, mittels der die Schwimmwanne (10) dem fallenden Wasserstand im Gefäß (1) nachführbar ist.

11. Vorrichtung nach Anspruch 10, dadurch gekennzeichnet, daß die Führung (113) einen am oberen Öffnungsrand (114) des Gefäßes (1) ansetzbaren Jochbügel (116) umfaßt, der ein Halteorgan (117) für eine die Schwimmwanne (10) führende Stange (118) aufweist.

12. Vorrichtung nach Anspruch 11, dadurch gekennzeichnet, daß die Schwimmwanne (10) eine auf ihrem Boden (109) über einer Bodenöffnung (119) stehende Hülse (120) aufweist, durch die die als Führung dienenden Stange (118) verläuft.

13. Vorrichtung nach einem der Ansprüche 9 bis 12, dadurch gekennzeichnet, daß an dem aus dem Boden (109) der Schwimmwanne (10) vorstehenden freien Ende der Stange (118) ein den Fallweg der Schwimmwanne (10) begrenzendes Anschlagorgan (121) angeordnet ist.

14. Vorrichtung zur Freisetzung von ätherischen Wirkstoffen aus geschnittenen Kräutern bzw. Kräutermischungen in einen abgegrenzten Raum zwecks Behandlung von Erkrankungen der Atemwege eines im Raum befindlichen Patienten,
**gekennzeichnet durch**
eine Wärmequelle (101) mit als Heizplatte (102) ausgebildeter waagerechter Stellfläche (3), durch ein auf die Stellfläche (103) stellbares Ständerteil (104) und durch ein vom Ständerteil (104) derart gehaltenes Kräuterreservoir (106), daß sich dessen Boden (109) in jeweils vorbestimmbaren Abständen über der Stellfläche (103) befindet.

15. Vorrichtung nach Anspruch 14, dadurch gekennzeichnet daß das Ständerteil (104) als Hohlzylinder ausgebildet ist.

16. Vorrichtung nach Anspruch 15, dadurch gekennzeichnet, daß das hohlzylindrische Ständerteil (104) an einem Ende eine nach Innen gerichtete, seinen freien Innenquerschnitt verengende Einziehung (105) aufweist.

17. Vorrichtung nach einem der Ansprüche 14 bis 16, dadurch gekennzeichnet, daß die Innenwand des hohlzylindrischen Ständerteils (104) als Distanzhalter wirkende Vorsprünge aufweist, die als radial nach Innen vorstehende Leiste (112) ausgebildet sind.

18. Vorrichtung nach einem der Ansprüche 14 bis 17, dadurch gekennzeichnet, daß das Kräuterreservoir (106) als Napf ausgebildet ist, der einen durch die Vorstehung der als Distanzhalter dienenden Leisten (112) des Ständerteils (104) vorbestimmten Außendurchmesser aufweist.

19. Vorrichtung nach Anspruch 18, dadurch gekennzeichnet, daß das Kräuterreservoir (104) am oberen Öffnungsrand radial vorstehende Halter (107) aufweist.

20. Vorrichtung nach Anspruch 19, dadurch gekennzeichnet daß als Halter (107) ein umlaufender Kragenflansch (108) vorgesehen ist, der trichterartig geneigt zum Öffnungsrand des Kräuterreservoirs (106) steht.

21. Vorrichtung nach einem der Ansprüche 14 bis 20, dadurch gekennzeichnet, daß das Kräuterreservoir (106) einen ebenen Boden (109) aufweist.

22. Vorrichtung nach einem der Ansprüche 14 bis 21, dadurch gekennzeichnet, daß in das Kräuterreservoir (106) einstellbare weitere Kräuteraufnahmen (110) vorgesehen sind.

23. Vorrichtung nach Anspruch 12, dadurch gekennzeichnet, daß jede Kräuteraufnahme (110) ein Hohlzylinder ist, der mit seinem unteren Rand auf den Boden (109) des Kräuterreservoirs (106) stellbar ist.

24. Vorrichtung nach einem der Ansprüche 22 und 23, dadurch gekennzeichnet, daß jede Kräuteraufrahme (110) an ihrem oberen Rand einen nach Außen vorstehenden Kragenflansch (111) aufweist, der trichterartig geneigt ist.

25. Vorrichtung nach einem der Ansprüche 14 bis 24, dadurch gekennzeichnet, daß der Werkstoff für das Ständerteil (104), das Krauterreservoir (106) und die Kräuteraufnahmen (110) Glas ist.

26. Vorrichtung nach einem der Ansprüche 14 bis 25, dadurch gekennzeichnet, daß die Wärmequelle (101) eine elektrische Haushaltswärmeplatte, insbesondere ein elektrisches Tee-Stövchen ist.
